# EUROPÄISCHE PATENTSCHRIFT

(11) **EP 1 259 205 B1**
(45) Veröffentlichungstag und Bekanntmachung des Hinweises auf die Patenterteilung: **17.11.2004**
(21) Anmeldenummer: 01917055.4
(22) Anmeldetag: 02.03.2001
(51) Int. Cl.: A61F 13/15, A61L 15/42, A61L 15/00

(54) **ABSORBIERENDE STRUKTUR SOWIE VERFAHREN ZU DEREN HERSTELLUNG**
ABSORBENT STRUCTURE AND METHOD OF PRODUCING THE SAME
STRUCTURE ABSORBANTE ET SON PROCEDE DE PRODUCTION

(30) Priorität: 02.03.2000 DE 10010268; 02.03.2000 DE 10010269
(43) Veröffentlichungstag der Anmeldung: 27.11.2002
(73) Patentinhaber: Paul Hartmann Aktiengesellschaft, 89522 Heidenheim (DE)
(72) Erfinder: MALOWANIEC, Krzysztof, D., 89522 Heidenheim (DE); MANGOLD, Rainer, 89542 Herbrechtingen (DE); WURSTER, Thomas, 89522 Heidenheim (DE)
(74) Vertreter: Friz, Oliver
(86) Internationale Anmeldenummer: PCT/EP2001/002385
(87) Internationale Veröffentlichungsnummer: WO 2001/064153

(56) Entgegenhaltungen:
- WO-A-98/56430
- GB-A- 2 151 272
- US-A- 5 061 259
- US-A- 5 859 077
- US-A- 6 019 871

## Beschreibung

Die Erfindung betrifft eine auf Basis körniger superabsorbierender Polymermaterialien gebildete absorbierende Struktur, wobei die superabsorbierenden Polymermaterialien durch ein niedriger schmelzendes thermoplastisches Polymer miteinander verbunden sind. Desweiteren betrifft die Erfindung ein Verfahren zum Herstellen einer derartigen absorbierenden Struktur und einen Hygieneartikel mit einer solchen absorbierenden Struktur als eine Saugkörperlage.

Wenn vorstehend von einer auf Basis superabsorbierender Polymermaterialien gebildeten absorbierenden Struktur die Rede ist, so wird hierunter eine Struktur mit einem Anteil von mehr als 70 Gewichtsprozent an superabsorbierenden Polymermaterialien verstanden.

Es hat sich gezeigt, dass Saugkörperstrukturen mit einem derart hohen Gehalt an superabsorbierenden Polymermaterialien (SAP) mit herkömmlichen auf Fasern basierenden Strukturen nicht realisierbar sind, da die körnigen SAP-Materialien einerseits nicht hinreichend zugänglich angeordnet und andererseits zugleich immobilisiert werden können.

Aus DE-A-2 222 780 ist es bekannt, zur Herstellung einer auf Basis superabsorbierender Polymermaterialen gebildeten absorbierenden Struktur die körnigen superabsorbierenden Polymermaterialien zusammen mit Teilchen eines thermoplastischen Werkstoffs auf eine Unterlage aufzubringen und den thermoplastischen Werkstoff dann zu erschmelzen, um einen Verbund zu erhalten.

Eine derartige Struktur aus superabsorbierenden Polymermaterialien und thermoplastischen Polymeren konnte sich aber in der Praxis nicht durchsetzen, da die Zugänglichkeit der superabsorbierenden Materialien für die auftreffende Flüssigkeit nicht hinreichend gewährleistet war Zudem erwies sich diese Struktur als zu starr und zeichnete sich daher durch einen ungenügenden Tragkomfort aus.

Hiervon ausgehend liegt der Erfindung die Aufgabe zugrunde, bei einer absorbierenden Struktur der eingangs genannten gattungsgemäßen Art mit hohem SAP-Gehalt die vorstehenden Nachteile zu beseitigen, d.h. eine flexible Struktur mit guter Flüssigkeitsaufnahme und -speichercharakteristik, die zudem einfach hergestellt werden kann, zu erreichen.

Diese Aufgabe wird durch eine absorbierende Struktur mit den Merkmalen des Anspruchs 1 gelöst.

Aus WO 94/13460 ist die Herstellung eines offenzelligen Polypropylenschaums mit einem Porenanteil von mehr als 20 Vol.-% bekannt. Als Anwendungsgebiete sind Verpackungszwecke und die Verwendung des Schaums zur Schallabsorption und thermischen Isolation genannt.

Aus WO 98/56430 ist ebenfalls die Herstellung eines extrudierten thermoplastischen Schaums bekannt. Der Schaum hat vorzugsweise eine aus Zellwänden und Hohlräumen bestehende Struktur. Der Schaum kann beschreibungsgemäß als Aufnahmekörper für ein Stück Fleisch oder als Lage einer Windel verwendet werden.

Mit der vorliegenden Erfindung wurde erstmals vorgeschlagen, körniges, partikelförmiges superabsorbierendes Polymermaterial durch zumindest teilerschmolzene thermoplastische Polymerwerkstoffe zu verbinden, indem die unter Druck und Temperatur stehende Mischung unter Zusatz eines Treibmittels extrudiert wird. Hierdurch kann einerseits eine Immobilisierung, d.h. eine Fixierung der körnigen superabsorbierenden Polymermaterialien innerhalb der Struktur erreicht werden, und andererseits wird eine Struktur gebildet, welche von einer auftreffenden wässrigen Flüssigkeit, wie z. B. Urin, sehr gut durchdrungen werden kann. Es zeigte sich, dass die Flüssigkeit sehr rasch in die durch Extrusion des Gemischs unter Expansion des Treibmittels gebildete offenporige geschäumte Struktur eindringen und zu den darin aufgenommenen superabsorbierenden Polymermaterialien gelangen kann, wo sie dann dauerhaft gespeichert wird. Es zeigte sich auch, dass die quellfähigen superabsorbierenden Polymermaterialien in der erfindungsgemäßen Struktur in weit geringerem Maße den Effekt des sogenannten Gelblockings verursachen, welcher sich bei höheren gewichtsprozentualen Anteilen von quellfähigen Polymermaterialien in absorbierenden Faserstrukturen als problematisch erweist, weil die in der Flüssigkeit quellenden Polymermaterialien die Faserzwischenräume zusammendrücken, so dass keine Kapillarität zum Flüssigkeitstransport in noch ungenutzte Saugkörperbereiche mehr zur Verfügung steht. Ein weiteres Problem bei aus natürlichen Fasern gebildeten Saugkörperstrukturen ist deren Neigung im eingenässten Zustand in sich zusammenzufallen, welches Problem auch als wet-collaps bezeichnet wird. Auch dies führt zu einer Reduzierung des Flüssigkeitsverteilungsvermögens innerhalb einer absorbierenden Struktur. Bei der erfindungsgemäßen extrudierten absorbierenden Struktur treten die vorstehend erörterten Probleme nicht oder in weit geringerem Maße auf, weshalb das Absorptionsvermögen der superabsorbierenden Polymermaterialien auch bei sehr hohen Konzentrationen von mehr als 70 Gew.% nahezu vollständig zur Aufnahme der auftreffenden Flüssigkeit zur Verfügung steht.

Die Korngröße der Partikel aus superabsorbierenden Materialien liegt im üblichen Bereich und beträgt im Massenmittel vorzugsweise etwa 200 - 800 µm, wobei vorzugsweise nicht mehr als 20 Masse-% der Partikel kleiner als 200 µm sind; es wird diesbezüglich auf die Offenbarung in der US-Patentschrift 5,061,259 verwiesen.

Die extrudierte offenporige Struktur weist eine Retentionskapazität von wenigstens 10g Flüssigkeit je Gramm der extrudierten Struktur auf. Die Absorptionskapazität kann in einem noch näher zu beschreibenden Testverfahren bestimmt werden.

In weiterer Ausbildung der Erfindung ist der masseprozentuale Anteil des thermoplastischen Polymers geringer als 20 Gew.%, insbesondere geringer als 10 Gew.% der absorbierenden Struktur.

Als thermoplastisches Polymer, welches quasi das Verbindungsmittel der superabsorbierenden körnigen Polymermaterialien bildet, hat sich in besonders bevorzugter Weise ein Polymer aus der Gruppe der Polyolefine, insbesondere Polypropylen und/oder Polyethylen, erwiesen. Auch entsprechende Copolymere, insbesondere Ethylenvinylacetatcopolymere sowie halogenierte Polyolefine sind verwendbar. Grundsätzlich sind jedoch auch andere thermoplastischer Polymere zur Herstellung der erfindungsgemäßen absorbierenden Struktur geeignet, z.B. solche aus der Gruppe der Styrolpolymere.

Um ein möglichst großes Flüssigkeitsaufnahmevolumen zur Verfügung zu stellen und eine möglichst große Oberfläche der superabsorbierenden Polymermaterialien zur Flüssigkeitsaufnahme zu exponieren, liegt der Schäumungsgrad bei wenigstens 50%, vorzugsweise ist er höher als 100%. Der Schäumungsgrad ist dabei definiert als die Volumenzunahme einer Masseeinheit der Mischung im Zustand innerhalb der Extrusionsvorrichtung einerseits bzw. im extrudierten Zustand der fertigen Struktur andererseits.

In vorteilhafter Weise kann die absorbierende Struktur zwischen 3 und 20, vorzugsweise zwischen 5 und 10 Gew.% Fasern als Zuschlagstoffe umfassen. Hierbei kann es sich um natürliche oder synthetische Fasern, vorzugsweise Polyesterfasern, handeln, deren Schmelz- oder Zersetzungstemperatur aber höher ist als die Schmelztemperatur des verwandten thermoplastischen Polymers innerhalb der Extrusionsvorrichtung. Die Fasern bewirken, daß beim Extrusionsvorgang Kanäle gebildet werden, die das Eindringen von wässriger Flüssigkeit in die Struktur fördern.

Die Erfindung ermöglicht in besonders vorteilhafter Weise, dass absorbierende Strukturen gebildet werden können, deren Flächengewicht in Längsrichtung und/oder in Querrichtung der Struktur variiert, wobei die Längsrichtung mit der Extrusionsrichtung übereinstimmt. Durch eine entsprechende Gestaltung einer Extrusionsöffnung, insbesondere eines Extrusionsschlitzes, lassen sich an sich beliebige Querschnittsstrukturen erzielen. So könnte insbesondere im Querschnitt senkrecht zur Längsrichtung betrachtet die Dicke der absorbierenden Struktur mittig größer sein und entsprechend der Struktur der Extrusionsöffnung zu den Seiten hin in beliebiger Weise abnehmen.

Wie auch alle nachfolgend zu erläuternden absorbierenden Strukturen kann die Struktur außerdem eine oberflächenaktive Substanz, insbesondere ein Hydrophilisierungsmittel zu einem Anteil von vzw. 0,2 - 10% umfassen. Die bereits extrudierte Struktur kann sekundär mit dem Hydrophilisierungsmittel beaufschlagt werden. Vorzugsweise wird dieses Mittel aber gemeinsam mit den übrigen Ausgangsstoffen dem Extruder zugeführt oder in die bereits erschmolzene Polymermasse injiziert, sie befindet sich also bereits in Mischung mit der Polymerschmelze bevor diese extrudiert wird.

Vorteilhafterweise werden hierfür Alkylsulfonate, Fettsäurederivate oder Fluorchemikalien - wie Sie in der Veröffentlichung "Polymer Melt Additives: Their Chemistry Structure and Uses", (Autoren Gasper et al. Vortrag während der Insight 1999 - Nonwovens Business/Fiber & Fabric Conferences, San Diego, California, 1-2 November 1999. Proceedings herausgegeben durch Marketing Technology Services, Inc.) beschrieben sind - eingesetzt.

Mit der vorliegenden Erfindung wird auch Schutz beansprucht für einen absorbierenden Hygieneartikel zum einmaligen Gebrauch, insbesondere eine Windel, eine Damenbinde oder eine Inkontinenzvorlage, mit einem insbesondere mehrschichtigen Saugkörper, der gekennzeichnet ist durch eine Saugkörperlage aus einer absorbierenden Struktur der vorstehend beschriebenen erfindungsgemäßen Art.

Diese Saugkörperlage kann auf der körperabgewandten Seite einer Flüssigkeitsverteiler- und Zwischenspeicherschicht angeordnet sein. Es ist auch denkbar, dass die Flüssigkeitsverteiler- und Zwischenspeicherschicht, die weniger oder überhaupt keine superabsorbierenden Polymermaterialien umfasst, ebenfalls als extrudierte geschäumte Struktur hergestellt ist. Solchenfalls könnten beide Saugkörperlagen innerhalb der Herstellungsmaschine durch Extrusion hergestellt und zur Bildung des Schichtenverbunds übereinandergelegt werden. Auch eine unmittelbare Coextrusion beider Schichten, d.h. Herstellung durch dieselbe Extrusionsvorrichtung, ist denkar und vorteilhaft.

Es ist darüberhinaus weiterhin möglich, die erfindungsgemäße absorbierende SAP-haltige Struktur selbst mehrschichtig auszubilden. So kann z. B. eine erste körperabgewandte Schicht von einer zweiten körperzugewandten Schicht überlagert sein. Solchenfalls kann z. B. die absorbierende SAP-haltige Struktur mit einem vorteilhaften SAP-Profil ausgestattet werden. Insbesondere kann die erste körperabgewandte Schicht weniger SAP (in Gew. % bezogen auf diese erste Schicht) enthalten als die zweite körperzugewandte Schicht. Dabei kann es vorteilhaft sein, dass die flächenhafte Erstreckung, das heißt Breite und/oder Länge der ersten körperabgewandten Schicht verschieden ist von der flächenhaften Erstreckung der zweiten körperzugewandten Schicht, insbesondere kann es vorteilhaft sein, die erste körperabgewandte Schicht hinsichtlich ihrer flächenhaften Erstreckung größer, insbesondere breiter auszubilden als die zweite körperzugewandte Schicht. Auch dieser mehrschichte Aufbau der absorbierenden SAP-haltigen Struktur selbst lässt sich einfach durch unmittelbare Coextrusion der Schichten herstellen.

Des weiteren wäre es denkbar, daß eine körperabgewandte flüssigkeitsundurchlässige Schicht, die üblicherweise von einer vorgefertigten Kunststoff-Folie gebildet ist, durch Coextrusion mit der Saugkörperlage hergestellt ist. In diesem Fall würde es sich als vorteilhaft und zweckmäßig erweisen, alle drei vorerwähnten Schichten oder gar noch weitere Schichten durch Coextrusion mittels einer einzigen Extrusionsvorrichtung innerhalb der Herstellungsmaschine auszubilden. Es kann dann vorteilhafterweise auf ein Fixiermittel, wie zum Beipiel ein Heißschmelzkleber, verzichtet werden, da die extrudierten Schichten untereinander aber auch gegenüber weiteren Lagen und/oder Elementen im Zuge ihrer Herstellung fixiert werden können.

Es wird generell angemerkt, daß auch die Flüssigkeitsverteiler- und Zwischenspeicherschicht, welche sehr wenig oder überhaupt keine superabsorbierenden Polymermaterialien enthält, im übrigen so ausgebildet und hergestellt werden kann, wie die erfindungsgemäße absorbierende Struktur bzw. die vorerwähnte Saugkörperlage. Sie kann also Zuschlagstoffe in Form von Fasern oder oberflächenaktive Substanzen aufweisen und beispielsweise mit variierender Dicke bzw. variierendem Flächengewicht hergestellt ausgebildet sein.

Wie bereits vorstehend angedeutet kann es sich als vorteilhaft erweisen, wenn der Saugkörper in Längsrichtung des Artikels oder in Querrichtung des Artikels eine variierende Dicke aufweist, d.h. wenn er profiliert ausgebildet ist. Durch eine Materialanhäufung in einem mittleren Bereich des Hygieneartikels kann demzufolge dort die zur Verfügung stehende Flüssigkeitsabsorptionskapazität mit an sich beliebigem Profil, insbesondere gaussförmig oder stufenförmig, gebildet werden.

Es ist aber in ganz besonders vorteilhafter Ausbildung der Erfindung auch möglich, dass die Saugkörperlage beidseits in Längsrichtung des Artikels verlaufende und in Richtung auf den Benutzer emporstehende Wandabschnitte aufweist, die eine Auslaufsperre bilden. Diese Wandabschnitte übernehmen dann die Funktion von in Richtung auf den Benutzer emporstehenden Bündchenelementen, die bei bekannten Hygieneartikeln üblicherweise aus Vliesstoffen mit eingebrachten Elastifizierungsmitteln gebildet sind.

Es versteht sich, dass derartige Wandabschnitte auch in Querrichtung des Artikels verlaufen können und auch dort eine Sperrwirkung, insbesondere zum Trennen von festen und flüssigen Körperausscheidungen, ausüben können.

Gegenstand der vorliegenden Erfindung ist auch ein Verfahren zum Herstellen einer absorbierenden Struktur, insbesondere nach den Ansprüchen 1 bis 10 mit folgenden Verfahrensschritten:
- Einbringen eines thermoplastischen Polymers in eine Extrusionsvorrichtung,
- Einbringen eines superabsorbierenden Polymermaterials in körniger Form in die Extrusionsvorrichtung, wobei der gewichtsprozentuale Anteil des superabsorbierenden Polymermaterials zu dem thermoplastischen Polymer wenigstens 70 Gew.% beträgt,
- Schmelzen des thermoplastischen Polymers bei Temperaturen unterhalb einer Schmelz- oder Zersetzungstemperatur des superabsorbierenden Polymermaterials,
- Einbringen eines Treibmittels unter Überdruck,
- Extrudieren des Gemischs, wobei das Treibmittel bei Druckabbau zur Schäumung des thermoplastischen Polymers führt, welches die körnigen Polymermaterialien miteinander matrixbildend verbindet.

Als Treibmittel wird vorzugsweise CO₂ verwendet, wobei gleichwohl auch gesättigte, ungesättigte, cyclische Kohlenwasserstoffe und halogenierte Kohlenwasserstoffe sowie Edelgase wie Argon, Helium, oder Stickstoff oder ein Wasser/Luft-Gemisch denkbar wäre.

Es wird vorzugsweise ein solcher Überdruck innerhalb der Extrusionsvorrichtung aufgebaut, dass das Treibmittel sich in einem sogenannten überkritischen Zustand befindet, in dem die Phasengrenze zwischen flüssigem und gasförmigem Aggregatzustand verschwindet und lediglich eine einzige homogene Phase vorliegt. Dieser Bereich liegt bei CO₂ bei Temperaturen oberhalb von etwa 31°C und Drücken oberhalb von etwa 73,5 bar vor. In diesem Zustand lässt sich das Treibmittel optimal zur Vorbereitung eines physikalischen Schäumungsvorgangs mit den superabsorbierenden Polymermaterialien und mit dem geschmolzenen thermoplastischen Polymer vermischen. Wird diese Mischung dann durch eine Extrusionsöffnung in einen Bereich niedrigeren Drucks gegeben, so verdampft das Treibmittel bei abnehmender Temperatur, und es entsteht die geschäumte offenporige Struktur.

Da aber nicht nur ein vorzugsweise überkritischer Zustand des Treibmittels erreicht werden muss, sondern auch das thermoplastische Polymer zumindest teilerschmolzen werden muss, werden innerhalb der Extrusionsvorrichtung Temperaturen von 80 bis 200 °C geschaffen.

In ganz besonders vorteilhafter Weiterbildung der Erfindung kann zum Herstellen der erfindungsgemäßen Struktur auch feuchtes superabsorbierendes Polymermaterial verwendet werden, dessen Feuchtigkeitsgehalt wenigstens 1 Gew.%, vorzugsweise wenigstens 4 Gew.% beträgt. Solchenfalls kann dann der Flüssigkeitsanteil zusätzlich als Treibmittel dienen.

Zur Herstellung von in Längs- und/oder Querrrichtung variierender Dicke oder Gestalt der herzustellenden Struktur wird der Extrusionsquerschnitt während des Extrudierens verändert. Wenn eine große Anzahl von entsprechend ausgebildeten Strukturen extrudiert werden soll, so erweist es sich als vorteilhaft, wenn der Extrusionsquerschnitt entsprechend oszillierend verändert wird. Dies erfolgt quer zur Extrusionsrichtung, und zwar in der Ablegerichtung, wodurch die Dicke einer extrudierten Bahn variiert wird, oder quer zur Ablegerichtung, wodurch deren Breite variiert wird.

Um die Zugänglichkeit der extrudierten Struktur für wässrige Flüssigkeiten zu erhöhen, ist es vorteilhaft, die extrudierte Struktur einer weiteren mechanischen Behandlung, z.B. einer Streckung, einer Verpressung (Walzung) und/oder einer Perforierung durch ein feines Nadelwerkzeug auszusetzen.

Vorteilhaft ist insbesondere eine mehrstufige Walzung der extrudierten Struktur. Eine mehrstufige Walzung ermöglicht die Anwendung mehrerer Temperatur- und/oder Druckstufen. Damit kann die extrudierte Struktur gezielter hinsichtlich der Erfordernisse ihrer späteren Verwendung verändert/optimiert werden. So hat es sich als vorteilhaft erwiesen, die extrudierte Struktur in einer ersten Kalanderstufe bei einer Temperatur zu verpressen, die geeignet ist, das thermoplastische Polymer in der extrudierten Struktur oberhalb des Einweichungspunktes zu halten. Je nach verwendetem Polymer hat sich eine Temperatur in der Kalanderstufe von 40-90°C, insbesondere 45-75°C, insbesondere 50-60°C als geeignet erwiesen. Vorteilhafterweise kann die extrudierte absorbierende Struktur anschließend in einer zweiten Kalanderstufe verpresst werden, die kalt, insbesondere bei Temperaturen von 0-30°C, insbesondere bei 15-25°C durchgeführt wird.

Es hat sich ferner als vorteilhaft erwiesen, außerdem eine Verstreckung der extrudierten Struktur vorzunehmen.

Als ganz besonders vorteilhaft erweist es sich, wenn das erfindungsgemäße Verfahren in einen Herstellungsprozess für Hygieneartikel integriert wird und dabei eine Saugkörperlage unmittelbar innerhalb einer Maschine extrudiert wird. Solchenfalls kann auf Faserbildungs- und Ablegestationen bei der Herstellungsmaschine (zumindest für die extrudierte Saugkörperlage) verzichtet werden. Wie bereits erwähnt, können auch mehrere Saugkörperlagen, die übereinander anzuordnen sind, innerhalb derselben Maschine hergestellt werden.

Weitere Einzelheiten, Merkmale und Vorteile der Erfindung ergeben sich aus den beigefügte Patentansprüchen sowie aus der zeichnerischen Darstellung und nachfolgenden Beschreibung einer Herstellungsvorrichtung, des Herstellungsverfahrens sowie einiger Ausführungsformen erfindungsgemäßer absorbierender Strukturen. In der Zeichnung zeigt:
- Figur 1: eine schematische Ansicht einer Vorrichtung zum Herstellen einer erfindungsgemäßen absorbierenden Struktur;
- Figuren 2 bis 6: verschiedene Ausführungsformen erfindungsgemäßer absorbierender Strukturen;
- Figur 7: eine weitere Ausführungsform einer mehrschichtigen erfindungsgemäßen absorbierenden Struktur und
- Figur 8: eine schematische Darstellung einer Coextrusionsvorrichtung.

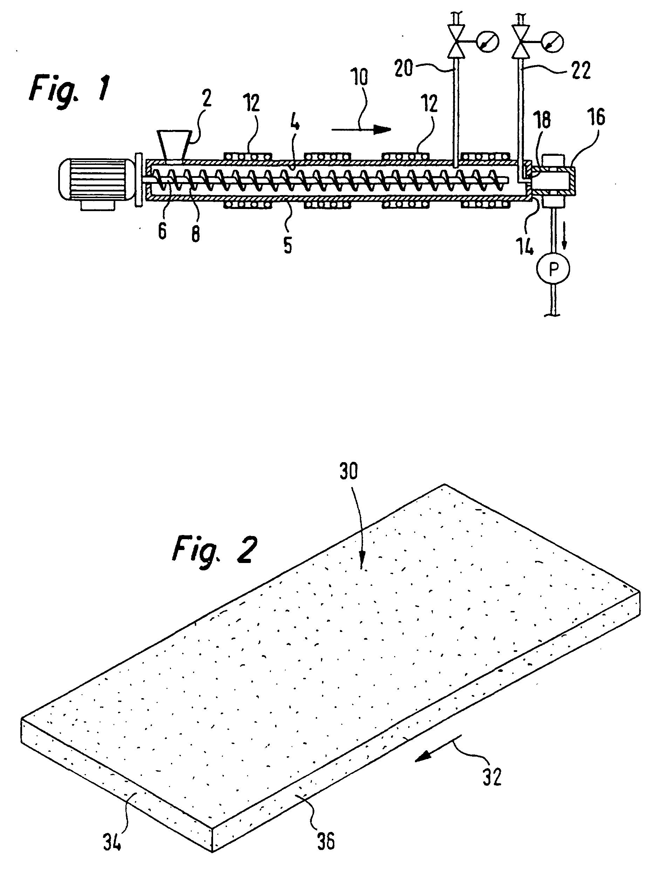

Figur 1 zeigt eine Vorrichtung zum Herstellen einer erfindungsgemäßen absorbierenden Struktur. Die Vorrichtung umfaßt eine trichterförmige Eingabeeinrichtung 2, über die ein Feststoffgemisch, das vorzugsweise zuvor gemäß der gewichtsprozentualen Zusammensetzung der einzelnen Bestandteile hergestellt wurde, in einen zylindrischen Innenraum 4 eines hochdruckstabilen rohrförmigen Gehäusekörpers 5 der Herstellungsvorrichtung eingegeben eingegeben werden kann. In diesen Innenraum 4 erstreckt sich eine elektromotorisch angetriebene Welle 6 mit einem wendelförmigen Schneckengang 8. Beim Antrieb der Welle 6 wird die eingebrachte Feststoffmischung weiter vermischt und in Längsrichtung 10 gefördert. Am Außenumfang des rohrförmigen Gehäuses 5 sind Heizeinrichtungen 12 vorgesehen.

An dem der Eingabeeinrichtung 2 gegenüberliegenden Ende des rohrförmigen Gehäuses 5 ist an dessen Stirnseite 14 ein Extrusionswerkzeug 16 montierbar. Das Extrusionswerkzeug 16 kommuniziert über eine Öffnung 18 an der Stirnseite 14 mit dem Innenraum 4 des rohrförmigen Gehäuses.

In den Innenraum 4 münden Injektionseinrichtungen 20, 22, wobei die letztere quasi innerhalb der Öffnung 18 mündet. Über die Injektionseinrichtungen 20, 22 kann ein unter Betriebsdruck stehendes Treibmittel in den Innenraum 4 eingebracht werden. Auf diese Weise kann im Innenraum 4 ein Betriebsdruck in Abhängigkeit des im Extrusionsvorgang verwandten Treibmittels, im allgemeinen oberhalb 70 bar, eingestellt und während des Extrusionsvorgangs aufrechterhalten werden.

Zur Herstellung einer erfindungsgemäßen absorbierenden Struktur kann beispielsweise als thermoplastisches Polymer ein Polyolefin, insbesondere ein Polypropylen- und/oder Polyäthylen-Granulat, verwendet werden. Dieses Granulat wird mit an sich bekannten quellfähigen superabsorbierenden Polymermaterialien, die in Verbindung mit absorbierenden Schichten bei Hygieneartikeln hinreichend bekannt sind und daher nicht näher beschrieben zu werden brauchen, vermischt. Das so erhaltene Gemisch wird über die Eingabeeinrichtung 2 in den Innenraum 4 gegeben. Durch die Heizeinrichtungen 12 wird das Gemisch auf eine solche Betriebstemperatur gebracht, daß das thermoplastische Polymer schmilzt, die körnigen superabsorbierenden Polymermaterialien aber in keinster Weise in Mitleidenschaft gezogen werden.

Über die erwähnten Injektionseinrichtungen 20, 22 wird ein Treibmittel, beispielsweise CO₂, in den Innenraum 4 eingeleitet, so daß dort ein Betriebsdruck herrscht, der zum Extrudieren des teilerschmolzenen Gemischs über das Extrusionswerkzeug 16 geeignet ist. Da das Treibmittel im Zuge der Extrusion zu einer Schäumung des thermoplastischen Polymers führen soll, wird es vorzugsweise im sogenannten "überkritischen Zustand" in den Innenraum 4 eingeleitet.

Beim Durchtritt der so erhaltenen Mischung durch die Extrusionsöffnung des Extrusionswerkzeugs 16 und durch den damit einhergehenden Druckabbau expandiert das Treibmittel und die Mischung wird geschäumt, d. h. es bilden sich durch das expandierende und in der Regel entweichende Treibmittel miteinander kommunizierende Poren oder Hohlräume. Innerhalb dieser durch Erstarren des thermoplastischen Polymers gebildeten Hohlraumstruktur sind die körnigen superabsorbierenden Polymermaterialien ortsfest gebunden. Sie sind immobilisiert, wobei dennoch ihre Oberfläche über die durch den Extrusionsvorgang und das Expandieren und Entweichen des Treibmittels gebildeten Hohlräume exponiert ist und zur Flüssigkeitsaufnahme zur Verfügung steht.

Figur 2 zeigt einen Abschnitt einer extrudierten absorbierenden Struktur 30, welche zu 80 Gew.% ein superabsorbierendes Polymermaterial und zu 13 Gew.% ein thermoplastisches Polymer, nämlich Polyethylen (PE), sowie zusätzlich zu 7 Gew.% Polyesterfasern (PES) umfasst.

Mit dem Pfeil 32 ist die Extrusionsrichtung bezeichnet, so daß die mit dem Bezugszeichen 34 gebildete Endfläche die Ebene senkrecht zur Extrusionsrichtung 32 darstellt. Die absorbierende Struktur 30 ist in Figur 2 exakt quaderförmig dargestellt; es wird darauf hingewiesen, daß durch einen Extrusionsvorgang nur eine im wesentlichen ebene Oberfläche erhalten werden kann, und daß auch bei exakt rechteckförmiger Extrusionsöffnung verrundete Kanten gebildet werden. Es wäre indessen möglich, eine in Extrusionsrichtung 32 endlose Bahn durch Längs- und Querschneiden mit exakt senkrecht zueinander verlaufenden Endflächen 34 und Längsseitenflächen 36 auszubilden.

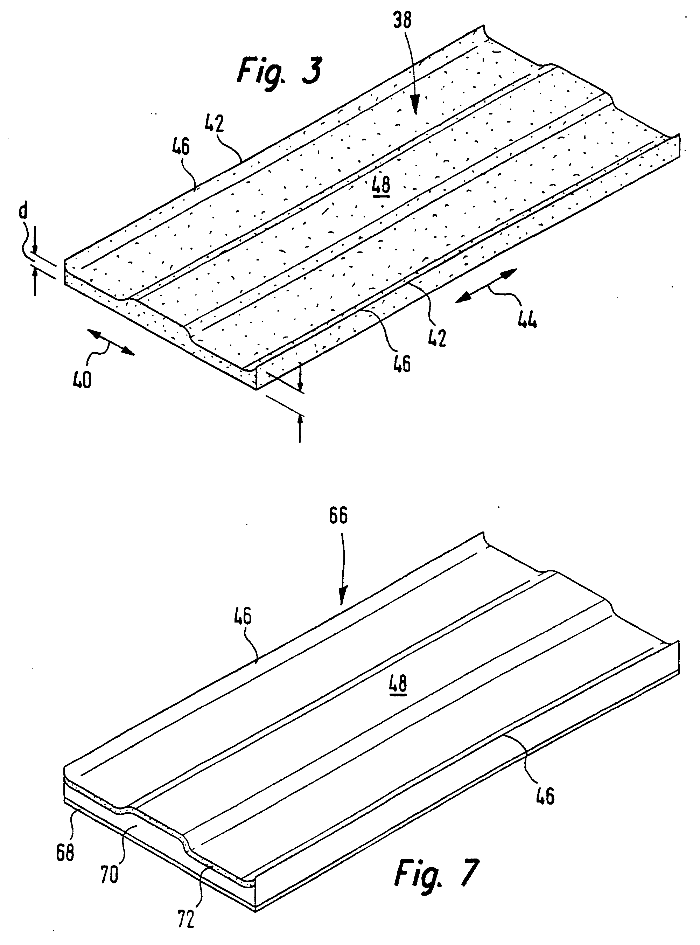

Figur 3 zeigt eine absorbierende Struktur 38, die in Querrichtung 40 eine variierende Dicke d aufweist. Die Struktur weist an ihren beiderseitigen Längsrändern 42 in Längsrichtung 44 verlaufend einen nach oben, also in Dickenrichtung emporstehenden Wandbereich 46 auf, der nach oben hin spitz ausläuft. Von außen nach innen, also in Querrichtung 40, fällt dieser Wandbereich 46 asymptotisch ab und geht in einen ebenen Abschnitt mit konstanter Dicke d über, um dann zur Mitte hin entsprechend dem aus Figur 3 ersichtlichen Profil zu einem Abschnitt 48 größerer Dicke wieder anzusteigen. Eine derartige Querschnittsstruktur läßt sich durch entsprechende Ausbildung des Extrusionsschlitzes herstellen.

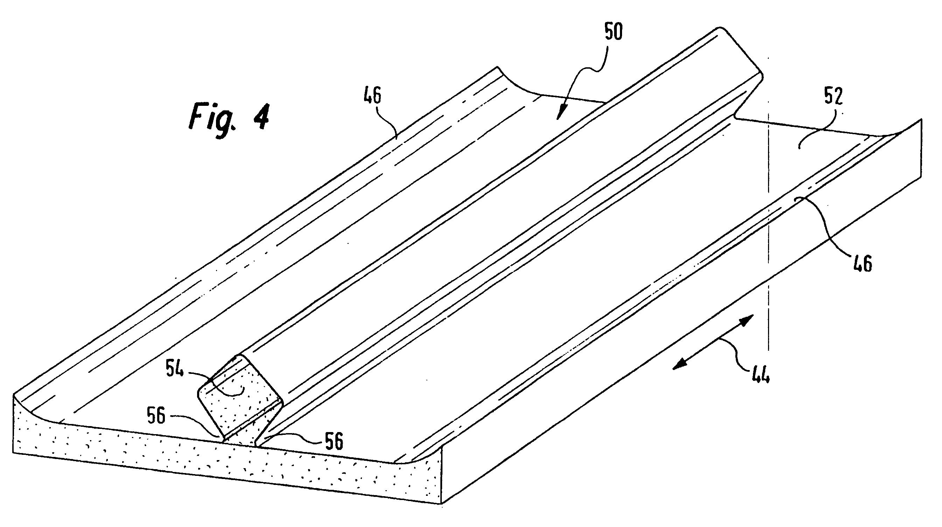

Figur 4 zeigt eine weitere Ausführungsform einer erfindungsgemäßen absorbierenden Struktur 50 mit entsprechend Figur 3 in Längsrichtung 44 verlaufenden emporstehenden beidseitigen Wandbereichen 46. Die Struktur 50 weist mittig einen ebenfalls in Längsrichtung 44 verlaufenden im Querschnitt im wesentlichen rautenförmigen und sich über einer Oberfläche 52 erhebenden Bereich 54 auf. Aufgrund seines rautenförmigen Querschnitts bildet der Bereich 54 in Richtung senkrecht zur Oberfläche 52 gesehen Hinterschnitte 56. Auch dies Ausbildung von im Querschnitt runden, elliptischen oder mehreckförmigen Strukturen mit oder ohne Hinterschnitte wäre denkbar. Derartige Saugkörperstrukturen sind zur Verwendung in Damenhygieneprodukten gedacht. Der erhabene Bereich 54, welche geometrische Form er auch immer haben mag, kann im Tragezustand zumindest teilweise in die Vagina eingreifen und somit einen direkten Kontakt zwischen Vagina und dem saugfähigen Hygieneprodukt herstellen.

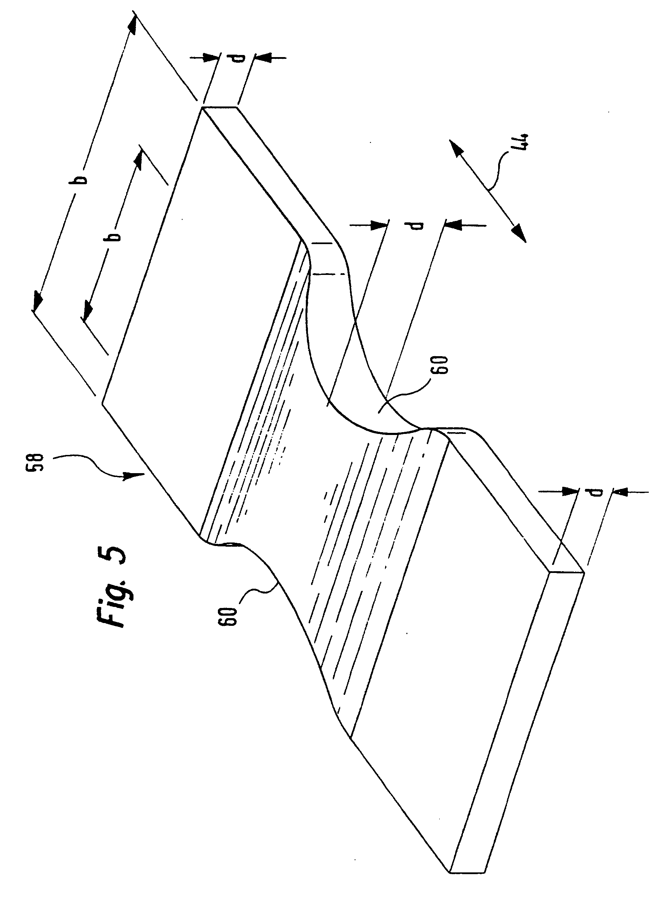

Figur 5 zeigt in entsprechender Ansicht eine durch Extrusion hergestellte absorbierende Struktur 58 mit in Längs- und Extrusionsrichtung 44 variierender Dicke d. Des weiteren weist die dargestellte absorbierende Struktur 58 in Längsrichtung 44 eine variierende Breite b auf. Die dargestellte absorbierende Struktur 58 würde sich zur Herstellung einer Windel eignen, wobei mittig bogenförmige Beinausschnitte 60 vorgesehen sind und in diesem den Schrittbereich der Windel bildenden Bereich eine Materialanhäufung durch die dort vorgesehene größere Dicke d gegeben ist.

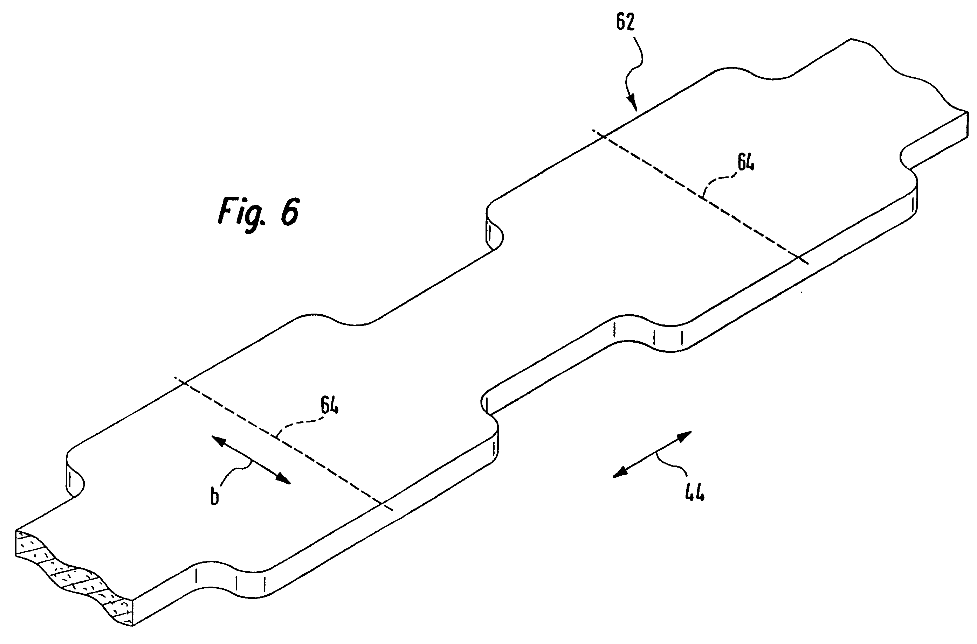

Figur 6 zeigt schematisch eine angedeutete endlose extrudierte Bahn 62 mit in Längs- und Extrusionsrichtung 44 variierender Breite b. Durch die unterbrochenen Linien 64 ist die Teilung der Endlosbahn durch Querschneiden zur Bildung einzelner Abschnitte für die Herstellung von Windeln angedeutet.

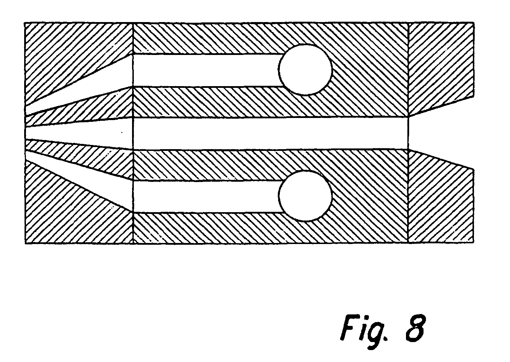

Figur 7 zeigt eine endlose extrudierte absorbierende Struktur 66, welche durch Coextrusion dreier Schichten hergestellt ist und sich für den Einsatz in einem Hygieneartikel, insbesondere einer Windel eignet. Die Struktur umfaßt eine erste untere extrudierte Folienschicht 68 aus PE und/oder PP. Eine mittlere auf Basis superabsorbierender Polymermaterialien gebildete extrudierte Schicht 70, die von der Zusammensetzung her der im Zusammenhang mit Figur 2 beschriebenen Schicht entsprechen kann, ist mit dem Bezugszeichen 70 bezeichnet. Auf deren Oberseite ist eine von superabsorbierenden Polymermaterialien freie Oberflächenschicht 72 auf Basis von Polyesterfasern (PES) einerseits und Polyethylen und/oder Polypropylen (PE/PP) andererseits vorgesehen. Alle drei Schichten 68; 70, 72 sind in einer Coextrusionsvorrichtung, wie sie schematisch in Figur 8 dargestellt ist, hergestellt, wobei zur Herstellung der Schichten 70 und 72 ein Treibmittel unter Überdruck eingesetzt wurde, um durch Expandieren und Verflüchtigen des Treibmittels eine offenporige geschäumte Struktur zu erzeugen. Die Struktur 66 ist im Querschnitt entsprechend Figur 3 ausgebildet; sie weist seitliche in Längsrichtung 44 verlaufende emporstehende Wandbereiche 46 auf, die in einem Hygieneartikel als Auslaufsperre dienen können und die Funktion von üblicherweise auf Basis von Vliesmaterialien gebildeten Bündchenelementen ausüben. Die Materialanhäufung durch eine größere Dicke der absorbierenden Schicht 70 in einem mittigen Bereich 48 stellt dort eine größere Flüssigkeitsabsorptionskapazität durch höhere Mengen an superabsorbierenden Polymermaterialien zur Verfügung. Die körperzugewandte obere Schicht 72 fungiert indessen als Flüssigkeitsverteiler- und Zwischenspeicherschicht. Dies bedeutet, sie erfaßt bei schwallartiger Flüssigkeitsbeaufschlagung eine große Menge von Flüssigkeit durch ihr großes Porenvolumen, um diese Flüssigkeit dann zeitverzögert in Dickenrichtung, aber auch in horizontaler Richtung zu verteilen und an die darunter befindliche Speicherschicht 70 abzugeben.

Das Flüssigkeitshaltevermögen einer erfindungsgemäßen extrudierten absorbierenden Struktur mit wenigstens 70 Gew.% Anteil an superabsorbierenden Polymermaterialien wird durch den nachfolgend zu beschreibenden Zentrifugentest durch Angabe des Retentionswerts bestimmt. Die zu untersuchende absorbierende Struktur wird im trockenen Zustand gewogen, um deren Masse in Gramm zu ermitteln. Es werden dann eine Anzahl von Prüflingen 30 Minuten lang vollständig in einer einprozentigen Natriumchlorid-Lösung von demineralisiertem Wasser als Prüflösung eingetaucht und anschließend 4 Minuten lang bei 276-facher Erdbeschleunigung geschleudert. Danach werden die Prüflinge wiederum gewogen, um die Masse einschließlich der darin gebundenen Flüssigkeit zu bestimmen. Die Masse der aufgenommenen oder gebundenen Flüssigkeit ergibt sich daher aus der Differenz der nach dem Schleudern bestimmten Masse und der Trockenmasse der jeweiligen Prüflinge. Dividiert man diese Differenz m_{fl} durch die Trockenmasse m_{trocken}, so erhält man den Retentionswert in der Einheit g_{fl}/g_{trocken}.

## Patentansprüche

1. Auf Basis superabsorbierender körniger, partikelförmiger Polymermaterialien gebildete absorbierende Struktur (30, 38, 50, 58, 62, 66) mit einem Anteil an superabsorbierenden Polymermaterialien von mehr als 70 Gew.-%, wobei die superabsorbierenden Polymermaterialien durch ein thermoplastisches Polymer miteinander verbunden sind, **dadurch gekennzeichnet, dass** sie ein unter Zusatz eines Treibmittels zu den superabsorbierenden Polymermaterialien und zu dem thermoplastischen Polymer geschäumtes offenporiges Extrusionsteil ist.

2. Absorbierende Struktur (30, 38, 50, 58, 62, 66), **dadurch gekennzeichnet, dass** die Struktur eine Rententionskapazität von wenigstens 10 g/g aufweist.

3. Absorbierende Struktur (30, 38, 50, 58, 62, 66) nach Anspruch 1 oder 2, **dadurch gekennzeichnet, dass** der gewichtsprozentuale Anteil des thermoplastischen Polymers an der absorbierenden Struktur geringer als 20 Gew.% ist.

4. Absorbierende Struktur (30, 38, 50, 58, 62, 66) nach Anspruch 3, **dadurch gekennzeichnet, dass** der gewichtsprozentuale Anteil des thermoplastischen Polymers geringer als 10 Gew.% der absorbierenden Struktur ist.

5. Absorbierende Struktur (30, 38, 50, 58, 62, 66) nach einem der vorstehenden Ansprüche, **dadurch gekennzeichnet, dass** das thermoplastische Polymer ein Polyolefin, insbesondere Polypropylen und/oder Polyethylen umfasst.

6. Absorbierende Struktur (30, 38, 50, 58, 62, 66) nach einem der vorstehenden Ansprüche, **dadurch gekennzeichnet, dass** der Schäumungsgrad größer als 50 % ist.

7. Absorbierende Struktur (30, 38, 50, 58, 62, 66) nach Anspruch 6, **dadurch gekennzeichnet, dass** der Schäumungsgrad größer als 100 % ist.

8. Absorbierende Struktur (30, 38, 50, 58, 62, 66) nach einem der vorstehenden Ansprüche, **dadurch gekennzeichnet, dass** die Struktur als Zuschlagstoffe 3 - 20 Gew.-% , insbesondere 5 - 10 Gew.-%, an Fasern umfasst.

9. Absorbierende Struktur (38, 50, 58, 62, 66) nach einem der vorstehenden Ansprüche, **dadurch gekennzeichnet, dass** deren Flächengewicht in Längsrichtung und/oder Querrichtung variiert.

10. Absorbierende Struktur (30, 38, 50, 58, 62, 66) nach einem der vorstehenden Ansprüche, **dadurch gekennzeichnet, dass** sie eine oberflächenaktive Substanz als Zuschlagstoff umfasst.

11. Absorbierender Hygieneartikel zum einmaligen Gebrauch, insbesondere Windel, Damenbinde, Inkontinenzvorlage, mit einem insbesondere mehrschichtigen Saugkörper, **gekennzeichnet durch** eine Saugkörperlage aus einer absorbierenden Struktur (30, 38, 50, 58, 62, 66) nach einem oder mehreren der vorstehenden Ansprüche.

12. Hygieneartikel nach Anspruch 11, **dadurch gekennzeichnet, dass** die Saugkörperlage (70) auf der körperabgewandten Seite einer Flüssigkeitsverteiler- und Zwischenspeicherschicht (72) angeordnet ist.

13. Hygieneartikel nach Anspruch 12, **dadurch gekennzeichnet, dass** die Flüssigkeitsverteiler- und Zwischenspeicherschicht (72) ein thermoplastisches Polymer umfasst und ein unter Zusatz eines Treibmittels zu dem thermoplastischen Polymer geschäumtes offenporiges Extrusionsteil ist.

14. Hygieneartikel nach Anspruch 13, **dadurch gekennzeichnet, dass** die Flüssigkeitsverteiler- und Zwischenspeicherschicht (72) keine superabsorbierenden Polymermaterialien umfasst.

15. Hygieneartikel nach Anspruch 13 oder 14, **dadurch gekennzeichnet, dass** die Flüssigkeitsverteiler- und Zwischenspeicherschicht (72) einen Schäumungsgrad von mehr als 50%, vorzugsweise von mehr als 100 % aufweist.

16. Hygieneartikel nach Anspruch 13, 14 oder 15, **dadurch gekennzeichnet, dass** die Flüssigkeitsverteiler- und Zwischenspeicherschicht (72) als Zuschlagstoff 1 - 20 Gew.-%, insbesondere 5 - 15 Gew.-% Fasern enthält.

17. Hygieneartikel nach einem der Ansprüche 11-16 mit einer auf der körperabgewandten Seite der Saugkörperlage (70) vorgesehenen flüssigkeitsundurchlässigen Folienschicht (68), **dadurch gekennzeichnet, dass** die Folienschicht (68) zusammen mit der Saugkörperlage (70) extrudiert ist und mit dieser ein einstückiges Extrusionsteil bildet.

18. Hygieneartikel nach einem der Ansprüche 11 - 17, **dadurch gekennzeichnet, dass** der Saugkörper in Längsrichtung (44) des Artikels eine variierende Dicke aufweist.

19. Hygieneartikel nach einem der Ansprüche 11 - 18, **dadurch gekennzeichnet, dass** der Saugkörper in Querrichtung (40) des Artikels eine variierende Dicke aufweist.

20. Hygieneartikel nach Anspruch 19, **dadurch gekennzeichnet, dass** die Saugkörperlage beidseits in Längsrichtung (44) des Artikels verlaufende und in Richtung auf den Benutzer emporstehende Wandabschnitte (46) aufweist, welche eine Auslaufsperre bilden.

21. Hygieneartikel nach einem der Ansprüche 11 - 20, **dadurch gekennzeichnet, dass** die Saugkörperlage einen in Querrichtung des Artikels verlaufenden und in Richtung auf den Benutzer emporstehenden Wandabschnitte aufweist.

22. Verfahren zum Herstellen einer absorbierenden Struktur nach einem oder mehreren der Ansprüche 1-10, die folgenden Verfahrensschritte umfassend:
- Einbringen eines thermoplastischen Polymers in eine Extrusionsvorrichtung,
- Einbringen eines superabsorbierenden körnigen Polymermaterials in die Extrusionsvorrichtung, wobei der gewichtsprozentuale Anteil des superabsorbierenden Polymermaterials zu dem thermoplastischen Polymer wenigstens 70 Gew.-% beträgt,
- Schmelzen des thermoplastischen Polymermaterials bei Temperaturen unterhalb einer Schmelz- oder Zersetzungstemperatur des superabsorbierenden Polymermaterials,
- Einbringen eines Treibmittels unter Überdruck,
- Extrudieren des Gemischs, wobei das Treibmittel bei Druckabbau zur Schäumung des thermoplastischen Polymers führt, welches die körnigen Polymermaterialien miteinander matrixbildend verbindet.

23. Verfahren nach Anspruch 22, **dadurch gekennzeichnet, dass** als Treibmittel CO₂ verwendet wird.

24. Verfahren nach Anspruch 22 oder 23, **dadurch gekennzeichnet, dass** das thermoplastische Polymer bei Temperaturen von 80 - 200 Grad Celsius erschmolzen wird.

25. Verfahren nach Anspruch 22, 23 oder 24, **dadurch gekennzeichnet, dass** superabsorbierende Polymermaterialien mit einem Feuchtigkeitsgehalt von wenigstens 1 Gew.-%, insbesondere von wenigstens 4 Gew.-%, verwendet werden.

26. Verfahren nach einem der Ansprüche 22 - 25, **dadurch gekennzeichnet, dass** als Zuschlagstoff Fasern in die Extrusionsvorrichtung eingebracht werden.

27. Verfahren nach einem der Ansprüche 22 - 26, **dadurch gekennzeichnet, dass** als Zuschlagstoff eine oberflächenaktive Substanz in die Extrusionsvorrichtung eingebracht wird.

28. Verfahren nach einem der Ansprüche 22 - 27, **dadurch gekennzeichnet, dass** ein Extrusionsquerschnitt während des Extrudierens verändert wird.

29. Verfahren nach Anspruch 28, **dadurch gekennzeichnet, dass** der Extrusionsquerschnitt oszillierend verändert wird.

30. Verfahren nach einem der Ansprüche 22 - 29, **dadurch gekennzeichnet, dass** das Verfahren in einen Herstellungsprozess für Hygieneartikel integriert wird und dabei die absorbierende Struktur unmittelbar innerhalb einer schnellaufenden Herstellungsmaschine für Hygieneartikel extrudiert wird.

31. Verfahren nach Anspruch 30, **dadurch gekennzeichnet, dass** innerhalb der schnellaufenden Herstellungsmaschine ein zweischichtiger Saugkörper durch Coextrusion der Schichten gebildet wird, wobei der Saugkörper die absorbierende Struktur (70) als Saugkörperlage und eine auf deren körperzugewandten Seite vorgesehene Flüssigkeitsverteiler- und Zwischenspeicherschicht (72) umfasst.

32. Verfahren nach Anspruch 31, **dadurch gekennzeichnet, dass** innerhalb der schnellaufenden Herstellungsmaschine ein dreischichtiger Saugkörper durch Coextrusion der Schichten gebildet wird, wobei die dritte Schicht eine flüssigkeitsundurchlässige Folienschicht (68) ist, die auf der körperabgewandten Seite der Saugkörperlage angeordnet ist.

## Claims

1. Absorbent structure (30, 38, 50, 58, 62, 66) formed on the basis of superabsorbent particular polymer materials with more than 70 weight-% of superabsorbent polymer materials, where the superabsorbent polymer materials are bonded by a thermoplastic polymer, **characterized in that** the structure is an extruded element which is open-pore foamed by addition of a blowing agent to the superabsorbent polymer and to the thermoplastic polymer.

2. Absorbent structure (30, 38, 50, 58, 62, 66), **characterized in that** the structure has a retention capacity of at least 10 g/g.

3. Absorbent structure (30, 38, 50, 58, 62, 66) according to claim 1 or 2, **characterized in that** the percentage by weight content of the thermoplastic polymer is less than 20% by weight of the absorbent structure.

4. Absorbent structure (30, 38, 50, 58, 62, 66) according to claim 3, **characterized in that** the percentage by weight content of the thermoplastic polymer is less than 10% by weight of the absorbent structure.

5. Absorbent structure (30, 38, 50, 58, 62, 66) according to one of the preceding claims, **characterized in that** the thermoplastic polymer comprises a polyolefin, especially polypropylene and/or polyethylene.

6. Absorbent structure (30, 38, 50, 58, 62, 66) according to one of the preceding claims, **characterized in that** the degree of foaming is more than 50%.

7. Absorbent structure (30, 38, 50, 58, 62, 66) according to claim 6, **characterized in that** the degree of foaming is greater than 100%.

8. Absorbent structure (30, 38, 50, 58, 62, 66) according to one of the preceding claims, **characterized in that** the structure comprises 3 - 20% by weight, especially 5 - 10% by weight, fibers as additives.

9. Absorbent structure (30, 38, 50, 58, 62, 66) according to one of the preceding claims, **characterized in that** its basis weight varies in the longitudinal and/or transverse direction.

10. Absorbent structure (30, 38, 50, 58, 62, 66) according to one of the preceding claims, **characterized in that** a surfactant substance is introduced as an additive.

11. Absorbent hygiene article for one-time use, especially diaper, feminine sanitary napkin, incontinence pad, having an especially multi-layer absorbent body, **characterized by** an absorbent body layer of an absorbent structure (30, 38, 50, 58, 62, 66) according to one or more of the preceding claims.

12. Hygiene article according to claim 11, **characterized in that** the absorbent body layer (70) is arranged on the side of a fluid distribution and interim retention layer (72) facing away from the body.

13. Hygiene article according to claim 12, **characterized in that** the fluid distribution and interim retention layer (72) comprises a thermoplastic polymer and is an extruded element which is open-pore foamed by addition of a blowing agent to the thermoplastic polymer.

14. Hygiene article according to claim 13, **characterized in that** the fluid distribution and interim retention layer (72) does not comprise any superabsorbent polymer materials.

15. Hygiene article according to claim 13 or 14, **characterized in that** the fluid distribution and interim retention layer (72) demonstrates a degree of foaming greater than 50%, preferably greater than 100%.

16. Hygiene article according to claims 13, 14 or 15, **characterized in that** the fluid distribution and intermediate retention layer (72) contains 1 - 20% by weight, especially 5 - 15% by weight fibers as an additive.

17. Hygiene article according to one of the claims 11 - 16 having a fluid-impermeable film layer (68) furnished on the side of the absorbent body layer (70), **characterized in that** the film layer (68) is extruded together with the absorbent body layer (70), and forms a one piece extrusion element with the absorbent body layer.

18. Hygiene article according to one of the claims 11 - 17, **characterized in that** the absorbent body has a varying thickness in the longitudinal direction (44) of the article.

19. Hygiene article according to one of claims 11 - 18, **characterized in that** the absorbent body varies in thickness in the transverse direction (40) of the article.

20. Hygiene article according to claim 19, **characterized in that** the absorbent body layer has wall sections (46) which form a leakage barrier and running on both sides in the longitudinal direction (44) of the article and projecting up toward the wearer.

21. Hygiene article according to one of claims 11 - 20, **characterized in that** the absorbent body layer has a wall section running basically in the transverse direction of the article and projecting up toward the wearer.

22. Method for producing an absorbent structure according to one or more of claims 1 - 10, comprising the following steps:
introduction of a thermoplastic polymer into an extrusion apparatus,
introduction of a superabsorbent granular polymer material into the extrusion apparatus, where the percentage by weight content of the superabsorbent polymer material to the thermoplastic polymer amounts to at least 70% by weight,
melting the thermoplastic polymer material at temperatures below a melting or degradation temperature of the superabsorbent polymer material,
introduction of a blowing agent under positive pressure,
extrusion of the mixture, whereby the blowing agent results in foaming of the thermoplastic polymer which bonds the granular polymer materials to form a matrix when pressure is reduced.

23. Method according to claim 22, **characterized in that** CO₂ is used as a blowing agent.

24. Method according to claim 22 or 23, **characterized in that** the thermoplastic polymer becomes molten at temperatures of 80 to 200 degrees Celsius.

25. Method according to claims 22, 23 or 24, **characterized in that** superabsorbent polymer materials with a moisture content of at least 1% by weight, especially of at least 4% by weight are used.

26. Method according to one of the claims 22- 25, **characterized in that** fibers are introduced into the extrusion apparatus as an additive.

27. Method according to one of the claims 22 - 26, **characterized in that** a surfactant substance is introduced into the extrusion apparatus as an additive.

28. Method according to one of the claims 22 - 27, **characterized in that** an extrusion cross section is changed during extrusion.

29. Method according to claim 28, **characterized in that** the extrusion cross section is changed in an oscillating fashion.

30. Method according to one of the claims 22 - 29, **characterized in that** the method is integrated into a production process for hygiene articles and therein the absorbent structure is extruded directly inside machinery for the high-speed production of hygiene articles.

31. Method according to claim 30, **characterized in that** a double-layer absorbent body is formed inside the high-speed production machinery by co-extrusion of the layers, wherein the absorbent body comprises the absorbent structure (70) as an absorbent body layer and a fluid distribution and intermediate retention layer (72) on the body-facing side of said element layer.

32. Method according to claim 31, **characterized in that** a triple-layer absorbent element is formed inside the high-speed production machinery by co-extrusion of the layers, wherein the third layer is a fluid-impermeable film (68) which is located on the side of the absorbent body layer facing away from the body.

## Revendications

1. Structure absorbante (30, 38, 50, 58, 62, 66) formée à base de matériaux polymères superabsorbants granuleux sous forme de particules et comportant une proportion de matériaux polymères superabsorbants plus grande que 70 % en poids, les matériaux polymères superabsorbants étant reliés entre eux au moyen d'un polymère thermoplastique, **caractérisée en ce qu'**elle est constituée par une pièce d'extrusion sous forme de mousse à pores ouverts avec apport d'un agent moussant aux matériaux polymères superabsorbants et au polymère thermoplastique.

2. Structure absorbante (30, 38, 50, 58, 62, 66), **caractérisée en ce que** la structure a une capacité de rétention égale à au moins 10 g/g.

3. Structure absorbante (30, 38, 50, 58, 62, 66) selon la revendication 1 ou 2, **caractérisée en ce que** la proportion en poids du polymère thermoplastique dans la structure absorbante est plus faible que 20 % en poids.

4. Structure absorbante (30, 38, 50, 58, 62, 66) selon la revendication 3, **caractérisée en ce que** la proportion en poids du polymère thermoplastique est plus faible que 10 % en poids de la structure absorbante.

5. Structure absorbante (30, 38, 50, 58, 62, 66) selon l'une quelconque des revendications précédentes, **caractérisée en ce que** le polymère thermoplastique comprend une polyoléfine, en particulier du polypropylène et/ou du polyéthylène.

6. Structure absorbante (30, 38, 50, 58, 62, 66) selon l'une quelconque des revendications précédentes, **caractérisée en ce que** le degré de moussage est plus grand que 50 %.

7. Structure absorbante (30, 38, 50, 58, 62, 66) selon la revendication 6, **caractérisée en ce que** le degré de moussage est plus grand que 100 %.

8. Structure absorbante (30, 38, 50, 58, 62, 66) selon l'une quelconque des revendications précédentes, **caractérisée en ce que** la structure comprend, en tant matières additionnelles, 3 à 20 % en poids, en particulier 5 à 10 % en poids de fibres.

9. Structure absorbante (30, 38, 50, 58, 62, 66) selon l'une quelconque des revendications précédentes, **caractérisée en ce que** son grammage varie dans la direction longitudinale et/ou dans la direction transversale.

10. Structure absorbante (30, 38, 50, 58, 62, 66) selon l'une quelconque des revendications précédentes, **caractérisée en ce qu'**elle comprend, en tant que matière additionnelle, une substance tensio-active.

11. Article d'hygiène à absorption à usage unique, en particulier couche pour bébé, serviette hygiénique, couche pour incontinent comportant un corps absorbant à plusieurs couches **caractérisé par** une couche de corps absorbant ayant une structure absorbante (30, 38, 50, 58, 62, 66) selon une ou plusieurs des revendications précédentes.

12. Article d'hygiène selon la revendication 11, **caractérisé en ce que** la couche de corps absorbant (70) est disposée sur la face opposée au corps de l'utilisateur d'une couche de répartition du liquide et de stockage intermédiaire (72).

13. Article d'hygiène selon la revendication 12, **caractérisé en ce que** la couche de répartition du liquide et de stockage intermédiaire (72) comprend un polymère thermoplastique et est constitué par une pièce d'extrusion sous forme de mousse à pores ouverts avec apport d'un agent moussant au polymère thermoplastique.

14. Article d'hygiène selon la revendication 13, **caractérisé en ce que** la couche de répartition du liquide et de stockage intermédiaire (72) ne comprend pas de matériaux polymères superabsorbants.

15. Article d'hygiène selon la revendication 13 ou 14, **caractérisé en ce que** la couche de répartition du liquide et de stockage intermédiaire (72) présente un degré de moussage plus grand que 50 %, de préférence plus grand que 100 %.

16. Article d'hygiène selon l'une quelconque des revendications 13, 14 ou 15, **caractérisé en ce que** la couche de répartition du liquide et de stockage intermédiaire (72) contient, en tant que matière additionnelle, de 1 à 20 % en poids, en particulier de 5 à 15 % en poids de fibres.

17. Article d'hygiène selon l'une quelconque des revendications 11 à 16, comportant une couche de feuilles imperméable aux liquides (68) qui est prévue sur la face opposée au corps de l'utilisateur de la couche de corps absorbant (70), **caractérisé en ce que** la couche de feuilles (68) est extrudée en même temps que la couche de corps absorbant (70) et forme une pièce d'extrusion en une pièce avec cette dernière.

18. Article d'hygiène selon l'une quelconque des revendications 11 à 17, **caractérisé en ce que** le corps absorbant présente une épaisseur qui varie dans la direction longitudinale (44) de l'article.

19. Article d'hygiène selon l'une quelconque des revendications 11 à 18, **caractérisé en ce que** le corps absorbant présente une épaisseur qui varie dans la direction transversale (40) de l'article.

20. Article d'hygiène selon la revendication 19, **caractérisé en ce que** la couche de corps absorbant (70) comporte, des deux côtés, des sections de paroi (46) qui s'étendent dans la direction longitudinale (44) de l'article, qui montent dans la direction de l'utilisateur et qui forment un barrage contre l'écoulement à l'extérieur.

21. Article d'hygiène selon l'une quelconque des revendications 11 à 20, **caractérisé en ce que** la couche de corps absorbant (70) comporte des sections de paroi qui s'étendent dans la direction transversale de l'article et qui montent dans la direction de l'utilisateur.

22. Procédé pour la fabrication d'une structure absorbante selon l'une quelconque des revendications 1 à 10 comprenant les étapes de procédé suivantes :
- introduction d'un polymère thermoplastique dans un dispositif d'extrusion,
- introduction d'un matériau polymère superabsorbant granuleux dans le dispositif d'extrusion, la proportion en poids du matériau polymère superabsorbant par rapport au polymère thermoplastique est au moins égale à 70 % en poids,
- fusion du matériau polymère thermoplastique à des températures qui sont inférieures à la température de fusion ou de décomposition du matériau polymère superabsorbant,
- introduction d'un agent moussant avec surpression,
- extrusion du mélange, l'agent moussant entraînant le moussage du polymère thermoplastique qui relie les matériaux polymère granuleux entre eux en formant une matrice.

23. Procédé selon la revendication 22, **caractérisé en ce que** l'on utilise du CO₂ en tant qu'agent moussant.

24. Procédé selon la revendication 22 ou 23, **caractérisé en ce que** le polymère thermoplastique est fondu à des températures comprises entre 80 et 100 degrés Celsius.

25. Procédé selon l'une quelconque des revendications 22, 23 ou 24, **caractérisé en ce que** l'on utilise des matériaux polymères superabsorbants ayant une teneur en humidité au moins égal à 1 % en poids, en particulier au moins égal à 4 % en poids.

26. Procédé selon l'une quelconque des revendications 22 à 25, **caractérisé en ce que** l'on introduit des fibres en tant que matière additionnelle dans le dispositif d'extrusion.

27. Procédé selon l'une quelconque des revendications 22 à 26, **caractérisé en ce que** l'on introduit une substance tensio-active en tant que matière additionnelle dans dispositif d'extrusion.

28. Procédé selon l'une quelconque des revendications 22 à 27, **caractérisé en ce que** l'on fait varier une section d'extrusion pendant le processus d'extrusion.

29. Procédé selon la revendication 28, **caractérisé en ce que** l'on fait varier la section d'extrusion de manière oscillante.

30. Procédé selon l'une quelconque des revendications 22 à 29, **caractérisé en ce que** le procédé est intégré dans un processus de fabrication pour des articles d'hygiène et que, dans ce cas, on extrude la structure absorbante directement à l'intérieur d'une machine de fabrication à grande vitesse pour des articles d'hygiène.

31. Procédé selon la revendication 30, **caractérisé en ce que**, à l'intérieur de la machine de fabrication à grande vitesse, on forme un corps absorbant à deux couches par extrusion en couches multiples (coextrusion), le corps absorbant comprenant la structure absorbante (70) en tant que couche de corps absorbant et une couche de répartition du liquide et de stockage intermédiaire (72) qui est prévue sur sa face dirigée vers le corps de l'utilisateur.

32. Procédé selon la revendication 31, **caractérisé en ce que**, à l'intérieur de la machine de fabrication à grande vitesse, on forme un corps absorbant à trois couches par extrusion en couches multiples (coextrusion), le corps absorbant comprenant la structure absorbante (70) en tant que couche de corps absorbant et une couche de répartition du liquide et de stockage intermédiaire (72) qui est prévue sur sa face dirigée vers le corps de l'utilisateur.
